Europäisches Patentamt

European Patent Office

Office européen des brevets

(11) Publication number: **0 193 228 B1**

# EUROPEAN PATENT SPECIFICATION

(45) Date of publication of patent specification: **02.11.89**

(21) Application number: **86200199.7**

(22) Date of filing: **11.02.86**

(51) Int. Cl.⁴: **C 07 D 303/24,**
C 07 D 301/02, C 07 C 93/06,
C 12 P 17/02

(54) Process for producing of 4-(2-methoxyethyl)-phenylglycidyl ether and/or metoprolol.

(30) Priority: **13.02.85 GB 8503666**

(43) Date of publication of application:
**03.09.86 Bulletin 86/36**

(45) Publication of the grant of the patent:
**02.11.89 Bulletin 89/44**

(84) Designated Contracting States:
**AT BE CH DE FR GB IT LI LU NL SE**

(56) References cited:
**FR-A-2 250 752**

**AGRICULTURAL & BIOLOGICAL CHEMISTRY,
vol. 43, no. 10, October 1979, pages 2099-2104,
Tokyo, JP; H. OHTA et al.: "Asymmetric
epoxidation of long chain terminal olefins by
Corynebacterium equi IFO 3730"**

**JOURNAL OF ORGANIC CHEMISTRY, vol. 46,
no. 15, 17th July 1981, pages 3128-3131,
American Chemical Society, Columbus, Ohio,
US; M.-J. de SMET et al.: "Practical approach
to high-yield enzymatic stereospecific organic
synthesis in multiphase systems"**

(73) Proprietor: **GIST-BROCADES N.V.
Wateringseweg 1 P.O. Box 1
NL-2600 MA Delft (NL)**
(73) Proprietor: **SHELL INTERNATIONALE
RESEARCH MAATSCHAPPIJ B.V.
Carel van Bylandtlaan 30
NL-2596 HR Den Haag (NL)**

(72) Inventor: **Phillips, Gareth Thomas
5 The Finches
Sittingbourne Kent (GB)**
Inventor: **Robertson, Brian William
1616 Lords Close Bapchild
Sittingbourne Kent, ME9 8AG (GB)**
Inventor: **Bertola, Mauro Attilio
A. van Scheltemaplein 91
NL-2624 PJ Delft (NL)**
Inventor: **Koger, Hein Simon
G. Vermootenstraat 32
NL-2064 XR Spaarndam (NL)**
Inventor: **Marx, Arthur Friedrich
Florence Nightingalelaan 12
NL-2614 GM Delft (NL)**
Inventor: **Watts, Peter Douglas
9 Woollett Road
Sittingbourne Kent (GB)**

**EP 0 193 228 B1**

(74) Representative: **Huygens, Arthur Victor, Dr. et al**
**c/o Gist-Brocades N.V. Patent & Trademarks**
**Department Wateringseweg 1 PO Box 1**
**NL-2600 MA Delft (NL)**

**Description**

The present invention relates to a process for the preparation of metoprolol in a stereospecific form or a pharmaceutically acceptable salt thereof like an acid addition salt, and/or a stereospecific form of 4 - (2 - methoxyethyl) - phenylglycidyl ether which comprises subjecting 4 - (2 - methoxyethyl) - phenylallyl ether to the action of a micro-organism having the ability for stereoselective epoxidation of 4 - (2 - methoxyethyl) - phenylallyl ether into 4 - (2 - methoxyethyl) - phenylglycidyl ether, having at least 80% by weight the S configuration, at least partly separating 4 - (2 - methoxyethyl) - phenylglycidyl ether and/or reacting 4 - (2 - methoxyethyl) - phenylglycidyl ether with isopropylamine and at least partly separating metoprolol and/or converting metoprolol into the pharmaceutically acceptable salt thereof.

It is commonly known that a lot of biologically active compounds exist as a mixture of stereoisomers. Most frequently these mixtures are used as such in agricultural and pharmaceutical applications. The major reason is that the separation costs still exceed the potential advantage of increase in activity. Usually the required biological activity resides in one stereoisomer so that at best the potency of the mixture is reduced to half. However it is apparent that modern pharmacologists are becoming increasingly aware of other implications of administering mixtures wherein one or more stereoisomers are regarded as an impurity that may not have the desired therapeutic effect but may well have other unwanted physiological effects including toxicity. Some examples are cited to illustrate the association of biological activity with single stereoisomers.

Within the pharmaceutical area most of the beta-adrenergic blocking agents are sold as mixtures although the activity resides in one stereoisomer. In one instance a drug known as labetalol has a combined alpha-adrenergic blocking and beta-adrenergic blocking action that has been shown to be attributed to two separate pairs of isomers from the mixture of four. N. Toda et al. reported in J. Pharmacol. Exp. Ther. *207* (1978) 311 that (−)-metoprolol is 270 to 380 times more potent than (+)-metoprolol in attenuating the response of rabbit atria and tracheal muscles to isoproterenol (a beta-adrenoreceptor stimulant).

Current routes to single stereoisomer beta-blockers, generally involve chemical resolutions or rather lengthy chemical syntheses from stereoisomeric active precursors which are for example described in US Patent 4.408.063 and in J. Org. Chem. *41* (1976) 3121 by L. M. Weinstok et al. Thus, these described processes to prepare analogously the S enantiomer (optically active stereoisomer) of metoprolol are not economically advantageous in industrial applications. Therefore an object of the present invention is to provide an efficient process for the preparation of such stereoisomers which may be carried out on an industrial scale in an economically attractive way.

The ability of micro-organisms to convert stereospecifically short-chain alkenes ($C_1$—$C_4$) into their corresponding epoxyalkanes in a gas/solid or in a two-liquid phase bio-reactor is demonstrated by J. Trampes et al. (3rd European Congress on Biotechnology, München, 10—14 September 1984). Another example of the microbiological preparation of epoxy-alkanes is disclosed in the U.S. Patent 4,106,986 describing the conversion of straight-chain 1-alkenes ($C_1$—$C_{20}$) into 1,2 - epoxyalkanes. In the European Patent Application 0099609 examples are given of the conversion of propene and 1-octene into their corresponding epoxyalkanes. However, the conversion of substituted alkenes, such as 4 - (2 - methoxyethyl) - phenylallyl ether, can in no way be deduced from these known processes, which are only applicable to straight or sometimes branched alkenes.

As a result of extensive research and experimentation an improved synthesis has now surprisingly been found for the preparation of particularly the S enantiomer of metoprolol and the S enantiomer of 4 - (2 - methoxyethyl) - phenylglycidyl ether, starting from 4 - (2 - methoxyethyl) - phenylallyl ether, using bacteria being active for the epoxidation of 4 - (2 - methoxyethyl) - phenylallyl ether to produce 4 - (2 - methoxyethyl) - phenylglycidyl ether which is showing at least 80% by weight the S configuration, whereafter at least partly 4 - (2 - methoxyethyl) - phenylglycidyl ether is separated and/or said 4 - (2 - methoxyethyl) - phenylglycidyl ether is reacted with isopropylamine to produce said metoprolol.

More specifically the present invention relates to a process for the preparation of metoprolol in a stereospecific form or a pharmaceutically acceptable salt thereof like an acid addition salt, and/or a stereospecific form of 4 - (2 - methoxyethyl) - phenylglycidyl ether which comprises subjecting 4 - (2 - methoxyethyl) - phenylallyl ether to the action of a bacterium having the ability in a suitable medium for stereoselective epoxidation of 4 - (2 - methoxyethyl) - phenylallyl ether into 4 - (2 - methoxyethyl) - phenylglycidyl ether, having at least 80% by weight the S configuration, at least partly separating 4 - (2 - methoxyethyl) - phenylglycidyl ether and/or reacting 4 - (2 - methoxyethyl) - phenylglycidyl ether with isopropylamine and at least partly separating metoprolol and/or converting metoprolol into the pharmaceutically acceptable salt thereof. Cross reference is made to EP—A—0 193 227 where the preparation of allyl ethers by stereoselective epoxidation using bacteria is described.

Preferably the process is carried out in such a way by selecting a proper micro-organism that of the metoprolol at least 90% by weight is formed in the S configuration.

With the term suitable micro-organisms is meant for example bacteria belonging to the genera *Rhodococcus, Myco-bacterium, Nocardia* and *Pseudomonas*. The micro-organisms are optionally immobilized with polymer gel. The micro-organisms for the epoxidation of 4 - (2 - methoxyethyl) - phenylallyl ether include cultures of species *Nocardia corallina* (an example of this species is deposited in the ATCC under the accession number of 31338), species *Rhodococcus sp.* (an example of this species is deposited in the NCIB under the accession number of 11277), species *Mycobacterium rhodochrous* (an example of this species is deposited in the NCIB under the accession number of 9703), species *Rhodococcus equi* (an example of this species is deposited in the NCIB under the accession number of 12035), species *Pseudomonas aeruginosa* (an example of this species is deposited in the NCIB under the accession number 12036), species *Pseudomonas oleovorans* (an example of this species is deposited in the ATCC under the accession number of 29347), species *Pseudomonas putida* (an example of this species is deposited in the NCIB under the accession number of 9571) and species *Pseudomonas aeruginosa* (an example of this species is deposited in the NCIB under the accession number of 8704).

In practising the preferred embodiment of the process of the present invention, a micro-organism having the ability to convert 4 - (2 - methoxyethyl) - phenylallyl ether into 4 - (2 - methoxyethyl) - phenylglycidyl ether, having at least 90% by weight the *S* configuration has to be selected from the above-mentioned micro-organisms, to be cultured for 0.5 to 10 days, whereafter the bacterial cells have to be collected from the culture solution, the cells are suspended in a liquid nutrient medium and 4 - (2 - methoxyethyl) - phenylallylether is subjected to the action of the cells.

The micro-organisms used in the present invention showing the epoxidation activity, have to be cultured for about 0.5 to 10 days, whereafter the cells are suspended in a liquid nutrient medium, preferably a minimal liquid nutrient medium, and 4 - (2 - methoxyethyl) - phenylallyl ether is subjected to the action of the cells. After the abovementioned cultivation for about 0.5 to 10 days, the cells may be isolated from the culturing medium before suspending the cells in the minimal liquid nutrient medium. To grow the micro-organisms used for the stereoselective epoxidation of 4 - (2 - methoxyethyl) - phenylallyl ether, ordinary culture mediums containing an assimilable carbon source (for example glucose, lactate, hydrocarbons like tetradecane (C14), etc.), a nitrogen source (for example ammonium sulphate, ammonium nitrate, ammonium chloride, etc.), with an agent for an organic nutrient source (for example, yeast extract, malt extract, peptone, meat extract, etc.) and an inorganic nutrient source (for example phosphate, magnesium, potassium, zinc, iron and other metals in trace amounts) may be used. Optionally an inducer (for example diethoxymethane) is added to the culture medium. A temperature between 0 and 45°C and a pH between 3.5 and 9 is maintained during the growth of the micro-organisms. Preferably the micro-organisms are grown at a temperature between 20 and 37°C and at a pH between 5 and 8.

The aerobic conditions required during the growth of the micro-organisms can be provided according to any of the well established procedures, provided that the supply of oxygen is sufficient to meet the metabolic requirement of the micro-organisms. This is most conveniently achieved by supplying a gaseous oxygen, preferably in the form of air. During the conversion of 4 - (2 - methoxyethyl) - phenylallyl ether into 4 - (2 - methoxyethyl) - phenylglycidyl ether the micro-organisms might be in a growing stage using an abovementioned ordinary culture medium. The micro-organisms may be supplemented with a cosubstrate.

Preferably during the conversion of 4 - (2 - methoxyethyl) - phenylallyl ether into 4 - (2 - methoxyethyl) - phenylglycidyl ether the micro-organisms can be held in a substantially non-growing stage using a minimal culture medium. As minimal culture medium, an ordinary culture medium may be used containing an assimilable carbon source when required (for example glucose, lactate, hydrocarbons like tetradecane (C14), etc.), a nitrogen source when required (for example ammonium sulphate, ammonium nitrate, ammonium chloride, etc.), with an agent for an organic nutrient source when required (for example yeast extract, malt extract, peptone, meat extract, etc.) and an inorganic nutrient source when required (for example phosphate, magnesium, potassium, zinc, iron and other metals in trace amounts). The micro-organisms can be kept in the non-growing stage for example under exclusion of the assimilable carbon source or under exclusion of the nitrogen source. A temperature between 0 and 45°C and a pH between 3.5 and 9 is maintained during this stage. Preferably the micro-organisms are kept at a temperature between 20 and 37°C and a pH between 5 and 8. The aerobic conditions required during this stage can be provided according to the abovementioned procedures, provided that the supply of oxygen is sufficient to meet the metabolic requirement of the micro-organisms but also to convert 4 - (2 - methoxyethyl) - phenylallyl ether into 4 - (2 - methoxyethyl) - phenylglycidyl ether. The 4 - (2 - methoxyethyl) - phenylglycidyl ether produced by the micro-organisms as mentioned above, can be recovered and purified according to any of the well established procedures. (−) - 4 - (2 - methoxyethyl) - phenylglycidyl ether, (+) - 4 - (2 - methoxyethyl) - phenylglycidyl ether or mixtures thereof produced according to the present invention can be converted into (+)-metoprolol, (−)-metoprolol or mixtures thereof, respectively, by the chemical reaction with isopropylamine. An example of the reaction to form beta-adrenergic receptor blocking agents with the absolute (S)-configuration from aryl glycidyl ethers with the absolute (S)-configuration is described in the German Patent Application 2453324.

Especially mixtures with a predominant amount of the compound (−)-metoprolol can be advantageously used in pharmaceutical products. Preferably those compounds having at least 80% and more preferably at least 90% by weight the *S* configuration can be used in pharmaceutical products.

An example for a pharmaceutically acceptable salt of metoprolol is: metoprolol tartrate prepared from metoprolol and L-tartaric acid. The optical purity as will be mentioned in the specification is represented as percent enantiomeric excess: S−R/S+R.

The present invention will be further described with reference to Examples in conjunction with the accompanying Figures, without restricting the scope of the present invention to these Examples.

Brief description of the Figures

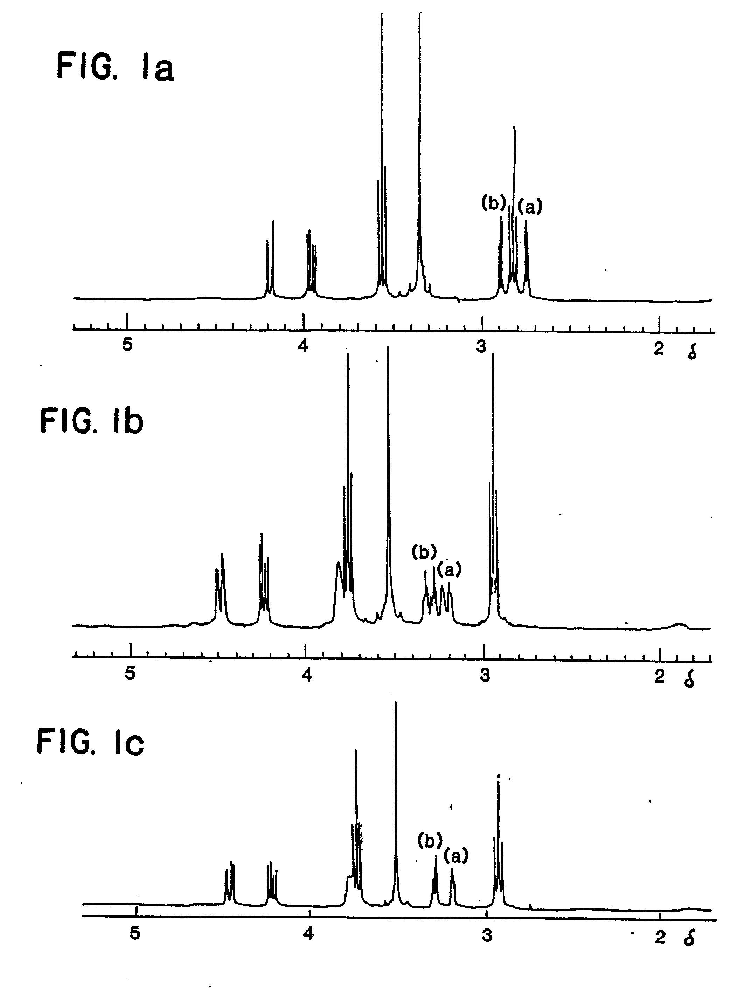

Figure 1 shows the partial pmr spectra of (+) and (±) - 4 - (2 - methoxyethyl) - phenylglycidyl ether in the presence of europium shift reagent.

(A): (±) or (+) - 4 - (2 - methoxyethyl) - phenylglycidyl ether without shift reagent.

B: (±) - 4 - (2 - methoxyethyl) - phenylglycidyl ether with $Eu(hfc)_3$; Ratio $Eu(hfc)_3/(±)$ - 4 - (2 - methoxyethyl) - phenylglycidyl ether=0.10.

C: (+) - 4 - (2 - methoxyethyl) - phenylglycidyl ether with $Eu(hfc)_3$; Ratio $Eu(hfc)_3/(+)$ - 4 - (2 - methoxyethyl) - phenylglycidyl ether=0.12.

Figure 2 shows the pmr spectrum of (−)-metoprolol.

Figure 3 shows the mass spectrum determined by C.I. ($CH_4$) of (−)-metoprolol.

Example I

Transformation of 4 - (2 - methoxyethyl) - phenylallyl ether into (+) - 4 - (2 - methoxyethyl) - phenylglycidyl ether by *Rhodococcus equi* NCIB 12035

Approximately half the biomass from a 72 h culture of *Rhodococcus equi* NCIB 12035, grown at 30°C on tetradecane (1 ml) in ASM mineral salts medium (100 ml), containing yeast extract (0.02%) was transferred to a conical flask (250 ml capacity) containing ASM (50 ml), yeast extract (to 0.02%), tetradecane (0.15 ml), octane (1 ml), Tween-80 (0.05 ml) and 4 - (2 - methoxyethyl) - phenylallyl ether (0.05 ml). The contents were incubated at 30°C, on an orbital shaker and samples were extracted into methylene chloride prior to analysis by gas chromatography on a Varian 3700. (Column: 3% OVI on WHP 100—120, 50 cm×2 mm i.d., 100°C—200°C at 10°C/min, $N_2$ at 30 cc/min). ASM contains $NH_4Cl$ (0.535 g/l), $KH_2PO_4$ (0.531 g/l), $Na_2HPO_4$ (0.866 g/l), $K_2SO_4$ (0.174 g/l), $MgSO_4 \cdot 7H_2O$ (0.037 g/l), $CaCl_2 \cdot 2H_2O$ (0.00735 g/l), $TK_3$ trace elements (1.0 ml/l), and $FeSO_4 \cdot 7H_2O$ (1.0 ml of a 0.1 M solution/l). $TK_3$ contains $ZnSO_4 \cdot 7H_2O$ (0.288 g/l), $MnSO_4 \cdot 4H_2O$ (0.224 g/l), $H_3BO_3$ (0.0618 g/l), $CuSO_4 \cdot 5H_2O$ (0.1248 g/l), $Na_2MoO_4 \cdot 2H_2O$ (0.0484 g/l), $CoCl_2 \cdot 6H_2O$ (0.0476 g/l), KI (0.083 g/l), 1 M $H_2SO_4$ (1 ml/l) at pH 7.0.

The product, (+) - 4 - (2 - methoxyethyl) - phenylglycidyl ether, appeared after 48 h incubation and increased until 96 h when the level of epoxide was approximately 15% of the remaining 4 - (2 - methoxyethyl) - phenylallyl ether.

In order to accumulate sufficient quantities of (+) - 4 - (2 - methoxyethyl) - phenylglycidyl ether, the culture broth from 10×50 ml and 5×500 ml incubations (conditions as described above) was extracted with methylene chloride (350 ml). The extract was dried over $Na_2SO_4$, the solvent evaporated, and the epoxide purified on silica gel using a hexane/ether gradient (epoxide eluted with 30% ether) to give (+) - 4 - (2 - methoxyethyl) - phenylglycidyl ether (353 mg) having $|\alpha|D^{25}=+8.11°$ (c=0.95, ethanol).

Optical purity of (+) - 4 - (2 - methoxyethyl) - phenylglycidyl ether was investigated using pmr in the presence of europium shift reagent $Eu(hfc)_3$ (see Figure 1). On addition of the shift reagent, the envelope of signals from each of the geminal protons (labelled (a) and (b)) in the chemically synthesised (±) - 4 - (2 - methoxyethyl) - phenylglycidyl ether (Figure 1A) shifted downfield and each was split into two envelopes of signals of equal intensity (Figure 1B, (a)+(b)). However, under the same conditions, only one envelope of signals was detectable from each of the geminal protons in the microbially produced (+) - 4 - (2 - methoxyethyl) - phenylglycidyl ether (Figure 1C, (a)+(b)). Thus, the optical purity of the (+) - 4 - (2 - methoxyethyl) - phenylglycidyl ether obtained by microbial epoxidation was determined to be 100%, within the experimental error of the pmr measurements.

Example II

Conversion of (+) - 4 - (2 - methoxyethyl) - phenylglycidyl ether into (−)-metoprolol

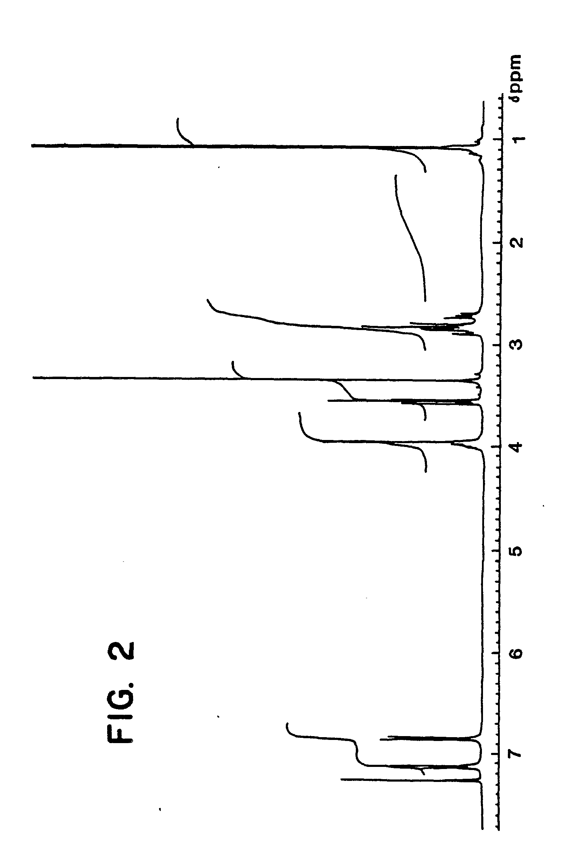

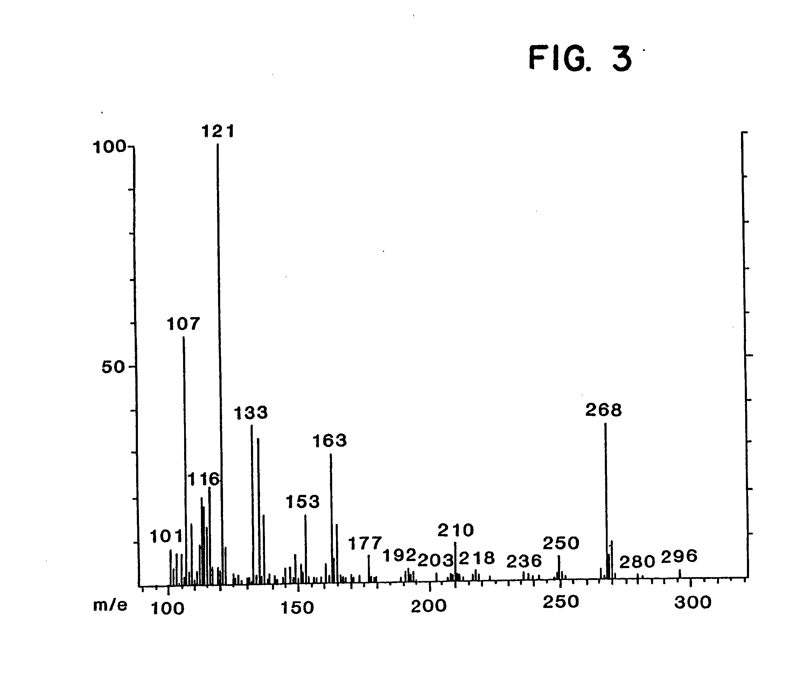

A solution of (+) - 4 - (2 - methoxyethyl) - phenylglycidyl ether (290 mg, see Example I) in dried ethanol (6.2 ml) containing isopropylamine (1.84 ml) was heated under reflux for 3 hours after which the solvents were removed by evaporation. The resulting oil was dissolved in methylene chloride (10 ml) and extracted into 0.2N HCl (10 ml) which was then washed with methylene chloride (2×10 ml). The acid layer was made basic with 2N NaOH (3 ml) and the product extracted into methylene chloride (10 ml). The methylene chloride extract was dried over $Na_2SO_4$ and the solvent evaporated to give a sticky solid. The product was recrystallized from hexane to give (−)-metoprolol (260 mg), showing $|\alpha|D^{25}=-5.46°$ (c=1.01, ethanol) and m.p. 42—45°C. (±)-Metoprolol m.p. 49—51°C, prepared chemically, was, as expected, optically inactive. The pmr and mass spectra of (−)-metoprolol (Figures 2—3) and (±)-metoprolol, (not shown), were consistent with the required structure, and were indistinguishable from the pmr spectrum of a sample of metoprolol tartrate.

Optical purity of the (−)-metoprolol was determined by separation of its S-leucyl diastereomeric amide derivatives on a reverse phase HPLC column (Lichrosorb RP8 (10 μm), 25 cm×1.4 inch o.d., 4.9 mm i.d., mobile phase: 40% acetonitrile in 0.1 M Na phosphate buffer pH 3.0 at 2.5 ml/min, U.V. detection). Derivatization was achieved by reacting metoprolol with the symmetrical anhydride of tertiary - butoxycarbonyl - S - leucine (BOS-S-leucine) followed by removal of the BOC group with trifluoracetic acid, as described by J. Hermansson and C. J. Von Bahr in J. Chrom., *227*, 113 (1982). Analysis of a sample

EP 0 193 228 B1

derived from the (±)-metoprolol resulted in two peaks of equal intensity, as expected for a racemate. The sample derived from the (−)-metoprolol gave two peaks with intensity ratio 97.7:2.3, equivalent to an optical purity of 95.4%.

Example III

Transformation of 4 - (2 - methoxyethyl) - phenylallyl ether into (+) - 4 - (2 - methoxyethyl) - phenylglycidyl ether by *Pseudomonas aeruginosa* strain 473 followed by its transformation into (−)-metoprolol

A suspension of *Pseudomonas aeruginosa* NCIB 12036 was prepared by resuspending cells (which had been grown in an ASM mineral salts medium containing 0.75% Na lactate and 0.05% diethoxymethane for 24 h at 30°C) in ASM to a volume equal to one tenth of the original culture volume.

Cell suspension (1100 ml) was incubated with 4 - (2 - methoxyethyl) - phenylallyl ether (3.3 g) at 30°C, 220 rpm. After 24 h, the reaction mixture was extracted with methylene chloride (500 ml), the extract dried over $Na_2SO_4$, and the solvent evaporated to give an oil (2.67 g). Purification on silica gel (as in Example I) gave (+) - 4 - (2 - methoxyethyl) - phenylglycidyl ether (930 mg) with $|a|D^{25}=+8.21°$ (c=0.943, ethanol).

Optical purity, as measured by pmr in the presence of europium shift reagent $Eu(hfc)_3$ (as in Example I), was determined to be 100% within the experimental error of the pmr measurements.

(+) - 4 - (2 - methoxyethyl) - phenylglycidyl ether (694 mg) was used (method as in Example II) to prepare (−)-metoprolol (579 mg) m.p. 44—46°C with $|a|D^{25}=-5.49°$ (c=1.02, ethanol). Optical purity was determined to be 98% (method as in Example II). The mother liquor was crystallised giving (−)-metoprolol (113 mg) with an optical purity of 96%.

Example IV

Transformation of 4 - (2 - methoxyethyl) - phenylallyl ether into (+) - 4 - (2 - methoxyethyl) - phenylglycidyl ether by *Pseudomonas aeruginosa* NCIB 8704 followed by its transformation into (−)-metoprolol

Cell suspension (200 ml) of *Pseudomonas aeruginosa* NCIB 8704 (preparation as described in Example III) was incubated with 4 - (2 - methoxyethyl) - phenylallyl ether (2 g) at 30°C, for 24 h after which the suspension was extracted and purified (as in Example III) to give (+) - 4 - (2 - methoxyethyl) - phenylglycidyl ether (91 mg). Optical purity of the epoxide, measured by pmr in the presence of europium shift reagent $Eu(hfc)_3$ (as in Example I) was determined to be 100% within the errors of the pmr measurements.

(+) - 4 - (2 - methoxyethyl) - phenylglycidyl ether (76 mg) was used (method as in Example II) to prepare (−)-metoprolol (55 mg), m.p. 42—45°C, $|a|D^{25}=-4.93°$ (c=0.944, ethanol), with an optical purity of 98.8% as determined by HPLC of its S-leucyl diastereomeric derivatives (as in Example II). Evaporation of the mother liquor yielded (−)-metoprolol (17 mg) of 95.2% optical purity.

Example V

Transformation of 4 - (2 - methoxyethyl) - phenylallyl ether into (+) - 4 - (2 - methoxyethyl) - phenylglycidyl ether by *Pseudomonas putida* NCIB 9571 followed by its transformation into (−)-metoprolol

Cell suspension (200 ml) of *Pseudomonas putida* NCIB 9571 (preparation as in Example III) was incubated with 4 - (2 - methoxyethyl) - phenylallyl ether (1 g) at 30°C for 24 hr, after which it was extracted into methylene chloride and purified (as in Example III) to give (+) - 4 - (2 - methoxyethyl) - phenylglycidyl ether (324 mg) with $|a|D^{25}=+6.42°$ (c=0.88, ethanol). Optical purity as measured by pmr in the presence of europium shift reagent $Eu(hfc)_3$ (as in Example I) was 100% within the experimental errors of the pmr measurements.

(+) - 4 - (2 - methoxyethyl) - phenylglycidyl ether (214 mg) was condensed with isopropylamine (as in Example II) to yield (−)-metoprolol (146 mg) m.p. 44—46°C with $|a|D^{25}=-5.00°$ (c=1.01, ethanol). Optical purity (method as in Example II) was determined to be 98%. Evaporation of the mother liquor yielded (−)-metoprolol (9 mg) of 97.5% optical purity.

Example VI

Transformation of 4 - (2 - methoxyethyl) - phenylallyl ether into (+) - 4 - (2 - methoxyethyl) - phenylglycidyl ether by *Pseudomonas oleovorans* ATCC 29347 followed by its transformation into (−)-metoprolol

Cell suspension (200 ml) of *Pseudomonas oleovorans* ATCC 29347 (preparation as in Example III) was incubated with 4 - (2 - methoxyethyl) - phenylallyl ether (2 g) at 30°C, for 5 h. Extraction into methylene chloride and purification on silica gel (as in Example I) yielded (+) - 4 - (2 - methoxyethyl) - phenylglycidyl ether (289 mg) with $|a|D^{25}=+8.02°$ (c=1.16, ethanol). Optical purity, as measured by pmr in the presence of europium shift reagent $Eu(hfc)_3$ (as in Example I) was determined to be 100% within the experimental errors of the pmr measurements.

(+) - 4 - (2 - methoxyethyl) - phenylglycidyl ether (171 mg) was reacted with isopropylamine (method as in Example II) to give (−)-metoprolol (154 mg) m.p. 44—45°C with $|a|D^{25}=-5.27°$ (c=0.976, ethanol). Optical purity was determined to be 98.4% based of HPLC on the S-leucyl diastereomeric amide

6

derivatives (as in Example II). Evaporation of the mother liquor gave (−)-metoprolol (6 mg) of 92.2% optical purity.

Example VII

Microbial transformation of 4 - (2 - methoxyethyl) - phenylallyl ether into (+) - 4 - (2 - methoxyethyl) - phenylglycidyl ether

Mineral salts medium (50 ml) containing Tween 80 (0.05 ml), tetradecane (0.05 ml) and 4 - (2 - methoxyethyl) - phenylallyl ether (0.05 ml) was inoculated with either *Nocardia corallina* ATCC 31338, *Rhodococcus sp.* NCIB 11277 or *Mycobacterium rhodochrous* NCIB 9703 (pregrown for 72 h on 0.5% tetradecane), then incubated at 30°C. Samples were taken at 24 h and 96 h, extracted into methylene chloride and analysed by gas chromatography (GC).

In each case, peaks corresponding to 4 - (2 - methoxyethyl) - phenylglycidyl ether were detected with conversions as follows: *Mycobacterium rhodochrous* 2% after 24 h; *Rhodococcus sp.* 1% after 96 h and *Nocardia corallina* 5% after 96 h. Further confirmation of the structure was obtained when the contents of a scaled up experiment using *Nocardia corallina* (500 ml of culture, conditions as above) were extracted into methylene chloride followed by purification on silica gel and pmr analysis. The pmr europium shift spectra of the 4 - (2 - methoxyethyl) - phenylglycidyl ether suggested an optical purity of 100%, and were identical to the europium pmr spectra of (+) - 4 - (2 - methoxyethyl) - phenylglycidyl ether obtained from *Rhodococcus equi*.

Example VIII

Transformation of 4 - (2 - methoxyethyl) - phenylallyl ether into 4 - (2 - methoxyethyl) - phenylglycidyl ether by *Pseudomonas oleovorans* ATCC 29347

A suspension of *Pseudomonas oleovorans* ATCC 29347 was prepared by resuspending cells (which had been grown to stationary phase in PSX mineral salts medium, pH 7, containing 0.75% glycerol and 0.05% diethoxymethane, at 30°C) in PSX, pH 7.5, to a volume equal to one tenth of the original culture volume.

Cell suspension (10 ml, dry wt 12.6 g/l, contained in a 250 ml conical flask) was incubated with 4 - (2 - methoxyethyl) - phenylallyl ether (250 µl) and glucose (50 mg) at 37°C on an orbital shaker. Formation of 4 - (2 - methoxyethyl) - phenylglycidyl ether was monitored by gas chromatography after extraction into methylene chloride (conditions as described in previous examples). The level of 4 - (2 - methoxyethyl) - phenylglycidyl ether in the incubation mixture was 7.30 g/l after six hours.

## Claims

1. A process for the preparation of metoprolol in a stereospecific form or a pharmaceutically acceptable salt thereof like an acid addition salt, and/or a stereospecific form of 4 - (2 - methoxyethyl) - phenylglycidyl ether which comprises subjecting 4 - (2 - methoxyethyl) - phenylallyl ether to the action of a bacterium having the ability in a suitable medium for stereoselective epoxidation of 4 - (2 - methoxyethyl) - phenylallyl ether into 4 - (2 - methoxyethyl) - phenylglycidyl ether, having at least 80% by weight the *S* configuration, at least partly separating 4 - (2 - methoxyethyl) - phenylglycidyl ether and/or reacting 4 - (2 - methoxyethyl) - phenylglycidyl ether with isopropylamine and at least partly separating metoprolol and/or converting metoprolol into the pharmaceutically acceptable salt thereof.

2. A process according to claim 1, wherein said micro-organism is able to convert 4 - (2 - methoxyethyl) - phenylallyl ether into 4 - (2 - methoxyethyl) - phenylglycidyl ether having at least 90% by weight the *S* configuration.

3. A process according to claims 1—2, wherein said bacterium belongs to the genus *Rhodococcus*, the genus *Mycobacterium*, the genus *Nocardia* or the genus *Pseudomonas*.

4. A process according to claim 3, wherein said bacterium is of the genus *Rhodococcus*.

5. A process according to claim 3, wherein said bacterium is of the genus *Mycobacterium*.

6. A process according to claim 3, wherein said bacterium is of the genus *Nocardia*.

7. A process according to claim 3, wherein said bacterium is of the genus *Pseudomonas*.

8. A process according to any of the preceding claims, wherein said bacterium is immobilized with polymer gel.

9. A process as claimed in claim 1, wherein the bacterium used is *Nocardia corallina*, preferably *Nocardia corallina* (ATCC 31338).

10. A process as claimed in claim 1, wherein the bacterium used is *Rhodococcus sp.*, preferably *Rhodococcus sp.* (NCIB 11277).

11. A process as claimed in claim 1, wherein the bacterium used is *Mycobacterium rhodochrous*, preferably *Mycobacterium rhodochrous* (NCIB 9703).

12. A process as claimed in claim 1, wherein the bacterium used is *Rhodococcus equi*, preferably *Rhodococcus equi* (NCIB 12035).

13. A process as claimed in claim 1, wherein the bacterium used is *Pseudomonas aeruginosa*, preferably *Pseudomonas aeruginosa* (NCIB 12036).

7

EP 0 193 228 B1

14. A process as claimed in claim 1, wherein the bacterium used is *Pseudomonas oleovorans*, preferably *Pseudomonas oleovorans* (ATCC 29347).

15. A process as claimed in claim 1, wherein the bacterium used is *Pseudomonas putida*, preferably *Pseudomonas putida* (NCIB 9571).

16. A process as claimed in claim 1, wherein the bacterium used is *Pseudomonas aeruginosa*, preferably *Pseudomonas aeruginosa* (NCIB 8704).

**Patentansprüche**

1. Verfahren zur Herstellung von Metoprolol in einer stereospezifischen Form oder einem pharmazeutisch unbedenklichen Salz davon, wie einem Säureadditionssalz, und/oder einer stereospezifischen Form von 4 - (2 - Methoxyethyl) - phenylglycidylether, dadurch gekennzeichnet, dass man 4 - (2 - Methoxyethyl) - phenylallylether der Einwirkung eines Bakteriums unterwirft, das in einem geeigneten Medium die Fähigkeit zur stereoselektiven Epoxidierung von 4 - (2 - Methoxyethyl) - phenylallylether zu 4 - (2 - Methoxyethyl) - phenylglycidylether hat, von dem mindestens 80 Gew.-% die S-Konfiguration haben, 4 - (2 - Methoxyethyl) - phenylglycidylether mindestens teilweise abtrennt und/oder 4 - (2 - Methoxyethyl) - phenylglycidylether mit Isopropylamin umsetzt und Metoprolol mindestens teilweise abtrennt und/oder Metoprolol in das pharmazeutisch unbedenkliche Salz davon überführt.

2. Verfahren nach Anspruch 1, dadurch gekennzeichnet, dass der Mikroorganismus 4 - (2 - Methoxyethyl) - phenylallylether in 4 - (2 - Methoxyethyl) - phenylglycidylether überzuführen vermag, von dem mindestens 90 Gew.-% die S-Konfiguration haben.

3. Verfahren nach den Ansprüchen 1 bis 2, dadurch gekennzeichnet, dass das Bakterium zu der Gattung *Rhodococcus*, der Gattung *Mycobacterium*, der Gattung *Nocardia* oder der Gattung *Pseudomonas* gehört.

4. Verfahren nach Anspruch 3, dadurch gekennzeichnet, dass das Bakterium zu der Gattung *Rhodococcus* gehört.

5. Verfahren nach Anspruch 3, dadurch gekennzeichnet, dass das Bakterium zu der Gattung *Mycobacterium* gehört.

6. Verfahren nach Anspruch 3, dadurch gekennzeichnet, dass das Bakterium zu der Gattung *Nocardia* gehört.

7. Verfahren nach Anspruch 3, dadurch gekennzeichnet, dass das Bakterium zu der Gattung *Pseudomonas* gehört.

8. Verfahren nach einem der vorangehenden Ansprüche, dadurch gekennzeichnet, dass das Bakterium mit Polymergel immobilisiert ist.

9. Verfahren nach Anspruch 1, dadurch gekennzeichnet, dass das verwendete Bakterium *Nocardia corallina*, vorzugsweise *Nocardia corallina* (ATCC 31338), ist.

10. Verfahren nach Anspruch 1, dadurch gekennzeichnet, dass das verwendete Bakterium *Rhodococcus sp.*, vorzugsweise *Rhodococcus sp.* (NCIB 11277), ist.

11. Verfahren nach Anspruch 1, dadurch gekennzeichnet, dass das verwendete Bakterium *Mycobacterium rhodochrous*, vorzugsweise *Mycobacterium rhodochrous* (NCIB 9703), ist.

12. Verfharen nach Anspruch 1, dadurch gekennzeichnet, dass das verwendete Bakterium *Rhodococcus equi*, vorzugsweise *Rhodococcus equi* (NCIB 12035), ist.

13. Verfahren nach Anspruch 1, dadurch gekennzeichnet, dass das verwendete Bakterium *Pseudomonas aeruginosa*, vorzugsweise *Pseudomonas aeruginosa* (NCIB 12036), ist.

14. Verfahren nach Anspruch 1, dadurch gekennzeichnet, dass das verwendete Bakterium *Pseudomonas oleovorans*, vorzugsweise *Pseudomonas oleovorans* (ATCC 29347), ist.

15. Verfahren nach Anspruch 1, dadurch gekennzeichnet, dass das verwendete Bakterium *Pseudomonas putida*, vorzugsweise *Pseudomonas putida* (NCIB 9571), ist.

16. Verfahren nach Anspruch 1, dadurch gekennzeichnet, dass das verwendete Bakterium *Pseudomonas aeruginosa*, vorzugsweise *Pseudomonas aeruginosa* (NCIB 8704), ist.

**Revendications**

1. Procédé de préparation du métoprolol sous une forme stéréospécifique ou d'un sel pharmaceutiquement acceptable de celui-ci tel qu'un sel d'addition d'acide et/ou d'une forme stéréospécifique de l'éther 4 - (2 - méthoxyéthyl)phénylglycidylique, qui comprend l'exposition de l'éther 4 - (2 - méthoxyéthyl)phénylallylique à l'action d'une bactérie ayant l'aptitude, dans un milieu approprié, à l'époxydation stéréosélective de l'éther 4 - (2 - méthoxyéthyl)phénylallylique en l'éther 4 - (2 - méthoxyéthyl)phénylglycidylique ayant la configuration *S* pour au moins 80% en poids, la séparation au moins partielle de l'éther 4 - (2 - méthoxyéthyl)phénylglycidylique et/ou la réaction de l'éther 4 - (2 - méthoxyéthyl)phénylglycidylique avec l'isopropylamine et la séparation au moins partielle du métoprolol et/ou la conversion du métoprolol en le sel pharmaceutiquement acceptable de celui-ci.

2. Procédé suivant la revendication 1, dans lequel le micro-organisme est apte à convertir l'éther 4 - (2 - méthoxyéthyl)phénylallylique en l'éther 4 - (2 - méthoxyéthyl)phénylglycidylique ayant la configuration *S* pour au moins 90% en poids.

8

3. Procédé suivant les revendications 1 et 2, dans lequel la bactérie appartient au genre *Rhodococcus*, au genre *Mycobacterium*, au genre *Nocardia* ou au genre *Pseudomonas*.

4. Procédé suivant la revendication 3, dans lequel la bactérie est du genre *Rhodococcus*.

5. Procédé suivant la revendication 3, dans lequel la bactérie est du genre *Mycobacterium*.

6. Procédé suivant la revendication 3, dans lequel la bactérie est du genre *Nocardia*.

7. Procédé suivant la revendication 3, dans lequel la bactérie est du genre *Pseudomonas*.

8. Procédé suivant l'une quelconque des revendications précédentes, dans lequel la bactérie est immobilisée par un gel de polymère.

9. Procédé suivant la revendication 1, dans lequel la bactérie utilisée est *Nocardia corallina*, de préférence *Nocardia corallina* (ATCC 31338).

10. Procédé suivant la revendication 1, dans lequel la bactérie utilisée est *Rhodococcus sp.*, de préférence *Rhodococcus sp.* (NCIB 11277).

11. Procédé suivant la revendication 1, dans lequel la bactérie utilisée est *Mycobacterium rhodochrous*, de préférence *Mycobacterium rhodochrous* (NCIB 9703).

12. Procédé suivant la revendication 1, dans lequel la bactérie utilisée est *Rhodococcus equi*, de préférence *Rhodococcus equi* (NCIB 12035).

13. Procédé suivant la revendication 1, dans lequel la bactérie utilisée est *Pseudomonas aeruginosa*, de préférence *Pseudomonas aeruginosa* (NCIB 12036).

14. Procédé suivant la revendication 1, dans lequel la bactérie utilisée est *Pseudomonas oleovorans*, de préférence *Pseudomonas oleovorans* (ATCC 29347).

15. Procédé suivant la revendication 1, dans lequel la bactérie utilisée est *Pseudomonas putida*, de préférence *Pseudomonas putida* (NCIB 9571).

16. Procédé suivant la revendication 1, dans lequel la bactérie utilisée est *Pseudomonas aeruginosa*, de préférence *Pseudomonas aeruginosa* (NCIB 8704).

FIG. Ia

FIG. Ib

FIG. Ic

FIG. 2

EP 0 193 228 B1

FIG. 3

EP 0 193 228 B1